# FASCICULE DE BREVET EUROPEEN

(11) **EP 1 420 758 B1**
(45) Date de publication et mention de la délivrance du brevet: **07.03.2007**
(21) Numéro de dépôt: 02796315.6
(22) Date de dépôt: 27.08.2002
(51) Int. Cl.: A61Q 19/00, A61K 8/02, A61K 8/34, A61K 8/73, A61K 8/81

(54) **COMPOSITION COSMETIQUE A EFFET TENSEUR DE LA PEAU**
KOSMETISCHE ZUSAMMENSETZUNG MIT HAUTSTRAFFENDER WIRKUNG
COSMETIC COMPOSITION WITH SKIN TIGHTENING EFFECT

(30) Priorité: 27.08.2001 FR 0111123
(43) Date de publication de la demande: 26.05.2004
(73) Titulaire: L V M H RECHERCHE, 45800 St. Jean de Braye (FR)
(72) Inventeur: DELAGE-GROUILLER, Hervé, F-94270 Le Kremlin Bicetre (FR); NOE, Brigitte, F-45000 Orleans (FR); MAHE, Christian, F-35000 Rennes (FR)
(74) Mandataire: Giraud, Françoise
(86) Numéro de dépôt international: PCT/FR2002/002941
(87) Numéro de publication internationale: WO 2003/017967

(56) Documents cités:
- EP-A- 0 614 914
- DATABASE WPI Week 199414 Derwent Publications Ltd., London, GB; AN 1994-115120 XP002205158 & JP 06 065048 A (KANEBO LTD. ET AL.), 8 mars 1994 (1994-03-08) cité dans la demande
- PATENT ABSTRACTS OF JAPAN vol. 14, no. 220 (C-717), 10 mai 1990 (1990-05-10) & JP 02 053707 A (H. NISHIDA), 22 février 1990 (1990-02-22) cité dans la demande
- PATENT ABSTRACTS OF JAPAN vol. 17, no. 327 (C-1073), 22 juin 1993 (1993-06-22) & JP 05 032523 A (POLA CHEM. IND.), 9 février 1993 (1993-02-09) cité dans la demande
- DATABASE WPI Week 199729 Derwent Publications Ltd., London, GB; AN 1997-318112 XP002205159 & RU 2 069 557 C (FITOLON CO), 27 novembre 1997 (1997-11-27) cité dans la demande
- DATABASE CA [en ligne] CHEMICAL ABSTRACTS SERVICE, COLUMBUS, OHIO, US; Database accession no. 1977:589311 XP002205157 cité dans la demande & JP 52 079034 A (POLA CHEMICAL INDUSTRY) 2 juillet 1977 (1977-07-02)
- S. BUDAVARI: "The Merck Index, 12th edition" 1996 , MERCK & CO , WHITEHOUSE STATION, N.J. XP002205155 * Page 45, # 240,241 *
- S. BUDAVARI: " The Merck Index, 12th edition" 1996 , MERCK & CO , WHITEHOUSE STATION, N.J. XP002205156 * Page 1490-1, # 8873 *
- DATABASE WPI Week 200222 Derwent Publications Ltd., London, GB; AN 2002-169257 XP002205160 & KR 2001 089 938 A (K. S. KIM), 17 octobre 2001 (2001-10-17)
- DATABASE WPI Week 198906 Derwent Publications Ltd., London, GB; AN 1989-042223 XP002232243 & JP 63 313709 A (LION CORP), 21 décembre 1988 (1988-12-21)

## Description

L'invention concerne essentiellement un mélange à effet tenseur de la surface de la peau, ainsi que son utilisation pour préparer une composition cosmétique à effet tenseur pour le lissage de la surface de la peau.

Il est connu par le document FR-A-2 478 468 une composition comprenant un mélange de deux fractions de hyaluronate de poids moléculaire différent, l'une ayant un faible poids moléculaire de l'ordre de 10 000 à 200 000 Daltons et l'autre un poids moléculaire élevé de l'ordre de 1 000 000 à 4 500 000 Daltons, dans un rapport déterminé pondéral compris entre 0,3 et 2, une protéine issue de la substance naturelle utilisée comme source de hyaluronate, ainsi que de l'eau en complément.

Cette composition peut également contenir de 0,05 à 5 % de sucre-alcool tel que le sorbitol, ainsi que de 0,2% à 1% de polysaccharide tel qu'un alginate. Ce document ne divulgue en aucun cas l'utilisation de produits à effet tenseur au sens de la présente invention. Il ressort de la description de cette demande de brevet, que la composition est décrite essentiellement comme ayant un effet hydratant.

Il est aussi connu par le document WO96/19180 l'utilisation dans une composition notamment pour raffermir la peau de polysaccharides associés à de la caséine. Lesdits polysaccharides sont utilisés à faible concentration comme agent filmogène. Ce document ne divulgue aucunement que la composition décrite possède un quelconque effet de lissage mécanique de la surface de la peau.

Il a été décrit dans la demande de brevet japonais JP 6065048 des compositions cosmétiques contenant à titre de constituants essentiels 25 à 80 % en poids d'alginate de sodium et 15 à 70 % d'alcool polyhydrique. Ces compositions présentent de bonne qualité d'adhésion à la peau et d'hydratation de celle-ci.

Il a maintenant été établi lors d'essais, réalisés par les inventeurs de la présente invention en vue de mettre au point un produit à effet tenseur de la surface de la peau, que, de façon surprenante, l'introduction d'alcool polyvinylique ou de polyvinylpyrrolidone dans des mélanges contenant un polysaccharide et du sorbitol permettait d'améliorer grandement les propriétés d'adhésion à la peau de ce mélange et que, au surplus, l'addition à ce mélange comprenant les trois constituants (polysaccharide, sorbitol et alcool polyvinylique et/ou polyvinylpyrrolidone) de cellulose ou d'un dérivé de cellulose, permettait d'éviter toute sensation de tiraillement de la peau, en conférant de l'élasticité à la matrice polymérique formée après application sur la peau de la composition, sans provoquer pour autant de relâchement.

Ceci a conduit les inventeurs de la présente invention à définir des nouveaux mélanges de produits présentant de remarquables propriétés cosmétiques et permettant de conférer à des compositions cosmétiques dans lesquelles ils se trouvent incorporés des propriétés d'effet tenseur sur la peau et d'adhésion à la peau, jamais observées à ce jour, propriétés qui sont liées à la présence simultanée dans le mélange d'un polysaccharide, de sorbitol et d'alcool polyvinylique ou de polyvinylpyrrolidone et éventuellement de cellulose ou de dérivés de cellulose, dans des proportions bien déterminées.

De l'étude documentaire réalisée par les inventeurs de la présente invention, il ressort que même si différents documents de littérature envisagent la présence de certains de ces constituants, voir même de leur ensemble, en combinaison, aucun ne révèle ou suggère les proportions particulières de ces constituants pour obtenir l'effet tenseur remarquable obtenu selon la présente invention.

Ainsi, le brevet japonais JP-2053707 décrit des compositions cosmétiques formant un film très fin sur la peau et destiné à atténuer les rides et à nourrir la peau. Les compositions décrites dans ce document contiennent essentiellement 0,2 % d'alcool polyvinylique, 2 % d'hydroxyméthylcellulose, 0,5 % d'alginate de sodium, 5 % d'alcool modifié, 4,96 % d'agent humidifiant et 87,34 % d'eau. Ce document ne suggère donc pas l'introduction d'un sucre.

Le brevet russe RU-2069557 décrit une crème hydratante et nettoyante contenant, entre autres constituants, de la méthylcellulose, ou de l'alginate de sodium et de la glycérine.

Cette composition ne présente aucun effet tenseur de la peau, ni aucun effet de lissage mécanique des rides.

Le brevet JP-5207934 décrit une composition cosmétique à teneur réduite en alcool polyvinylique nourrissant la peau. Cette composition contient de l'alginate en tant qu'agent épaississant, à des teneurs qui ne permettent pas d'obtenir l'effet tenseur recherché selon la présente invention.

La demande japonaise JP-5032523 décrit des compositions cosmétiques contenant un polymère hydrosoluble, qui peut contenir, entre autres de la cellulose, ou de l'alginate de sodium, ainsi qu'éventuellement de l'alcool polyvinylique. Ce produit est décrit comme ayant un effet absorbant des huiles et comme apportant une sensation de fraîcheur et n'a aucun effet tenseur de la peau.

La demande coréenne publiée sous le numéro KR-2001089938 a été publiée le 17 octobre 2001 et décrit des compositions cosmétiques destinées à humidifier et blanchir la peau. Ces compositions contiennent 3,4 % d'alcool polyvinylique, 16,6 % de sorbitol, 4,7 % de glycérine, 30,2 % de zéolithe, 3,6 % de talc, 0,14 % de chitosan, 0,26 % d'alginate de sodium et 41,1 % d'extraits naturels de plante dans l'alcool. Les concentrations très faibles en polysaccharide des compositions décrites dans ce document ne permettent en aucun cas d'obtenir l'effet tenseur recherché selon la présente invention.

La présente invention permet de fournir un nouveau mélange à effet tenseur de la surface de la peau, pour la préparation d'une composition cosmétique présentant un effet tenseur de manière contrôlée, et permettant un lissage visible et durable de la surface de la peau avec un très grand confort d'utilisation.

La présente invention a encore pour but de résoudre le nouveau problème technique consistant en la fourniture d'un nouveau produit, notamment à effet tenseur de la surface de la peau, capable d'agir efficacement pour réaliser un lissage de la surface de la peau en obtenant notamment un effacement total ou partiel des rides, y compris les rides moyennes et profondes.

La présente invention permet de résoudre pour la première fois ce nouveau problème technique de manière satisfaisante, sûre et fiable, utilisable à l'échelle industrielle et cosmétique.

Ainsi, l'invention concerne selon une de ses caractéristiques essentielles un mélange à effet tenseur de la peau constitué de :
- 10 à 80 % en poids d'un polysaccharide ayant un poids moléculaire inférieur à 600 000 Daltons, de préférence inférieur à 400 000 Daltons et de préférence encore inférieur à 200 000 Daltons,
- 15 à 75 % en poids de sorbitol,
- 3 à 15 % en poids d'alcool polyvinylique ou de polyvinylpyrrolidone,
- 0 à 25 % en poids de cellulose ou d'un dérivé de cellulose, tel que la carboxyméthylcellulose ou l'hydroxyméthylcellulose.

Avantageusement, le polysaccharide précité est une pectine, un carraghénane, un galactomannane, un xanthane ou un alginate.

Selon un mode de réalisation préféré, le polysaccharide est un alginate, en particulier un alginate de propylène glycol, ou un alginate sous forme d'acide ou de sel, en particulier un alginate de sodium.

Parmi les alginates commerciaux utilisables dans les compositions selon la présente invention, on peut citer ceux de type Manugel.

Selon un autre mode de réalisation particulièrement avantageux de l'invention, le polysaccharide précité est un alginate se présentant sous la forme d'un extrait d'algue, en particulier un extrait d'algue de la famille des Laminariacae, de préférence un extrait d'algue de Laminaria digitata, de Laminaria flexicaulis, ou encore de Laminaria saccharina. Il existe des extraits d'algues commerciaux qui conviennent à la mise en oeuvre de l'invention. Ils sont notamment commercialisés par la Société IdB Holding comme par exemple : Laminaria digitata extract, Laminaria saccharina extract, Laminarine, ou encore Phytelene of Laminaria EG 483 liquid et EG 749.

Selon une variante de réalisation avantageuse de l'invention, la concentration en polysaccharide est comprise entre 20 et 55% en poids par rapport au poids total dudit mélange.

Selon une variante de l'invention, le sorbitol se présente sous la forme d'un extrait de plante, en particulier un extrait de sorbes, tel qu'un extrait de sorbes de Sorbus aucuparia, de sorbes de Sorbus domestica ou de sorbes de Sorbus aria.

Selon une autre variante de réalisation avantageuse de l'invention, la concentration en sorbitol est comprise 40 et 65 % en poids par rapport au poids total dudit mélange.

Selon un mode de réalisation particulièrement préféré de invention, le mélange à effet tenseur de la surface de la peau contient un extrait de Laminaria flexicaulis et un extrait de sorbes, en particulier un extrait de Sorbus aucuparia.

Selon un mode de réalisation particulièrement avantageux de l'invention, la concentration en polysaccharide est supérieure à la concentration en sorbitol.

Selon ce mode de réalisation, il a été observé que la concentration plus importante en polysaccharide permet d'obtenir un aspect mat après application d'une composition contenant ledit mélange à effet tenseur.

Selon un autre mode de réalisation particulièrement avantageux de l'invention, la concentration en sorbitol est supérieure à la concentration en polysaccharide. Il a été observé que, lorsque la concentration en sorbitol est supérieure à la concentration en polysaccharide, on obtient un aspect relativement brillant après application d'une composition contenant ledit mélange à effet tenseur.

Selon encore une autre caractéristique avantageuse de ce mode de réalisation de l'invention, la concentration en alcool polyvinylique (APV) ou en polyvinylpyrrolidone (PVP) est comprise entre 3 et 10% en poids par rapport au poids total dudit mélange.

Suivant une caractéristique avantageuse, le mélange contient de la cellulose ou l'un de ses dérivés, tel que de préférence de la carboxyméthylcellulose (CMC), et ce, à une concentration comprise entre 1 et 8% en poids par rapport au poids total dudit mélange.

Il a été constaté que l'APV agit en synergie avec le sorbitol, pour procurer une meilleure adhésion à la peau de la composition cosmétique définie plus loin, et d'autre part que l'APV en présence de CMC évite la sensation de « tiraillement » de la peau, en conférant de l'élasticité à la matrice polymérique formée après application sur la peau de ladite composition, sans provoquer pour autant de relâchement.

En effet, la composition cosmétique définie plus loin, préparée à partir dudit produit, forme après application sur la peau une matrice polymérique. Le fluage se définit comme la propriété de la matrice polymérique de se déformer sous l'action d'une contrainte. Quant au relâchement ou relaxation, il se définit comme la propriété de la matrice polymérique de perdre de la force de tension lorsqu'elle subit une déformation. Le terme plastifiant se réfère à des qualités d'assouplissement et d'élasticité de la matrice polymérique.

Ainsi, il a été constaté qu'en jouant sur les proportions relatives des quatre constituants essentiels du mélange de l'invention, il était possible de jouer non seulement sur l'effet tenseur mais également de conférer à ce mélange une action anti-fluage, anti-relâchement et/ou plastifiante plus ou moins marquée.

Les mélanges préférés selon l'invention sont constitués d'alginate, de sorbitol, d'APV ou de PVP et de CMC dans les proportions suivantes :
- 10 à 80 %, de préférence 20 à 55 %, en poids d'alginate,
- 15 à 75 %, de préférence 40 à 65 %, en poids de sorbitol,
- 3 à 15 %, de préférence 3 à 10 %, en poids d'alcool polyvinylique ou de polyvinylpyrrolidone,
- 0 à 25 %, de préférence 1 à 8 %, en poids de carboxyméthylcellulose.

Selon un deuxième aspect, l'invention concerne l'utilisation du produit à effet tenseur tel que défini précédemment pour la préparation d'une composition cosmétique à effet tenseur en vue d'obtenir un lissage mécanique de la surface de la peau.

On prépare généralement à cet effet un mélange intermédiaire comprenant de 5 à 15 %, de préférence 8 à 10 %, du mélange selon l'invention dans un excipient aqueux classique.

Plus précisément, l'invention concerne de nouvelles compositions cosmétiques contenant le mélange de l'invention.

Selon un mode préféré de réalisation de l'invention, le mélange de l'invention est incorporé dans ladite composition cosmétique à une concentration comprise de 0,2 à 90 %, de préférence de 0,4 à 70 %, de préférence encore de 0,4 à 5 % et plus préférentiellement de 1 à 3 %, en poids par rapport au poids total de ladite composition d'un mélange.

Suivant une variante de l'invention, ladite composition est caractérisée en ce qu'elle contient en outre au moins un agent biologiquement actif tel qu'un agent hydratant, un agent anti-ride, un agent anti-oxydant, un agent anti-radicalaire, un agent réparateur des effets destructeurs des rayons ultraviolets ou un agent amincissant tel que la caféine.

Suivant un mode préféré de réalisation de cette variante de l'invention, ladite composition est caractérisée en ce qu'elle contient en outre un parfum, une matière colorante cosmétiquement acceptable et/ou un agent protecteur des rayons ultraviolets UVA et UVB, notamment un filtre ou un nano pigment, tel qu'un oxyde de zinc ou de titane.

Selon divers modes de réalisation particuliers, ladite composition cosmétique est caractérisée en ce qu'elle peut se présenter sous la forme d'un gel, d'une lotion, d'un sérum, d'une suspension, d'une émulsion, huile-dans-eau ou eau-dans-huile, ou sous forme solide, par exemple sous forme d'un stick ou d'un patch, ou sous forme sèche, par exemple sous forme d'une poudre.

Pour réaliser une forme sèche sous forme de poudre, on pourra par exemple vaporiser, dans un mélangeur, sur une poudre cosmétique classique le mélange selon l'invention préalablement dissous dans un excipient aqueux. Bien entendu, la quantité de solution de mélange vaporisée sera compatible avec la nécessité du maintien de la forme poudre. Généralement, cette quantité n'excédera pas 5%. Suivant une autre méthode d'incorporation du mélange selon l'invention dans une forme sèche, celui-ci pourra être fixé sur l'excipient de la poudre, tel que du talc, par exemple au moyen d'un lit d'air fluidisé.

La composition selon l'invention forme une pellicule, qui présente des propriétés mécaniques de type élastomère avec une texture qui se prête particulièrement bien au maquillage et notamment à l'utilisation de poudres qui s'y fixent particulièrement bien. De plus ce type de texture possède des propriétés anti-migratrices qui peuvent s'avérer utiles tant sur le plan esthétique que sur celui du confort d'utilisation notamment à proximité de zones sensibles comme par exemple les yeux.

Selon un troisième aspect, la présente invention concerne encore un procédé de soin cosmétique, caractérisé en ce qu'il comprend l'application topique sur les zones de la peau concernées d'une quantité efficace d'une composition cosmétique, telle que définie précédemment, pour obtenir un effet de lissage de la surface de la peau.

Avantageusement, ce procédé est aussi caractérisé en ce que, ultérieurement à l'application de la composition cosmétique telle que définie précédemment, et en cas de diminution de l'effet tenseur de la surface de la peau, on réalise une humidification des zones de la peau concernées ayant reçu au moins une première application de ladite composition cosmétique, afin de restaurer ledit effet tenseur, notamment par pulvérisation d'eau ou d'une préparation aqueuse cosmétiquement acceptable.

Grâce à l'action mécanique de l'effet tenseur du mélange, tel que précédemment défini, incorporé dans une composition cosmétique, on obtient un effacement partiel ou total des rides et en particulier des rides moyennes et profondes. Autrement dit, on réalise un lissage de la surface de la peau, visible, progressif, et durable. Ladite composition cosmétique permet également de remédier à de nombreuses imperfections présentes à la surface de la peau. Elle a en effet une action non négligeable sur la réduction de la visibilité de certaines imperfections couramment dénommées « peau d'orange », des vergetures, de certaines cicatrices, ainsi que de certaines zones du corps qui présentent habituellement des plis comme par exemple le cou.

L'invention présente encore un autre avantage en comparaison avec les compositions antérieures à effet tenseur. A la différence de ces dernières, la composition selon l'invention présente un effet tenseur progressif, ce qui en pratique se traduit par une absence d'effet dit « de pic ». L'effet de pic, pour les compositions antérieures à effet tenseur, conduit au tiraillement de la peau, et même, le cas échéant, à la rupture et/ou au décollement de la matrice polymérique. Cet effet de pic peut donc engendrer une perte brutale et soudaine de l'effet tenseur de la surface de la peau.

On remarquera encore que les bonnes qualités présentées par la composition selon l'invention quant à la résistance au fluage et au relâchement de la matrice polymérique déjà mentionnées, permettent notamment d'utiliser des quantités plus importantes d'actifs, de charges ou de conservateurs par exemples.

Par ailleurs, le mélange selon l'invention permet d'augmenter la pénétration des actifs dans la peau, ce qui a pour effet d'améliorer leur biodisponibilité.

Ainsi, l'invention concerne également l'utilisation des mélanges selon l'invention comme agent cosmétique pour favoriser la pénétration dans la peau d'un agent biologiquement actif.

Ainsi, le mélange selon l'invention permet d'augmenter la quantité d'agents biologiquement actifs pénétrant dans la peau par rapport à une quantité donnée de ces agents appliqués en surface. Ceci a pour effet d'améliorer leur biodisponibilité, autrement dit d'augmenter la quantité disponible de ces actifs dans les couches inférieures de la peau, et ainsi de contribuer à augmenter leur efficacité.

Plus précisément, le mélange selon l'invention, en particulier lorsqu'il a été introduit dans la formule d'une composition cosmétique contenant un agent biologiquement actif, permet d'obtenir dans un premier temps une accumulation dudit agent biologiquement actif dans le stratum corneum, appelée couramment "effet réservoir", et dans un deuxième temps, une libération progressive et prolongée de cet actif vers les couches de la peau plus profondes. En comparaison, une composition cosmétique identique à l'exception qu'elle ne contiendrait pas le mélange selon l'invention, ne présenterait pas cet effet réservoir et donc ne permettrait pas la pénétration d'une aussi grande quantité d'actif dans la peau.

Ainsi grâce à la mise en oeuvre de la présente invention, il est notamment possible d'obtenir un double effet: réaliser un effet tenseur de la peau, tout en obtenant un traitement biologique amélioré, en particulier pour atténuer ou retarder biologiquement l'apparition des rides, pour améliorer ou restaurer l'hydratation de la peau, pour atténuer les taches pigmentaires cutanées ou encore pour obtenir un effet amincissant.

D'autres buts, caractéristiques et avantages de l'invention apparaîtront clairement à partir de la description suivante donnée à partir d'exemples illustratifs et non limitatifs de l'invention représentant des modes de réalisation actuellement préférés. Dans les exemples, tous les pourcentages sont donnés en poids, la température est la température ambiante, la température est donnée en degrés Celsius et la pression est la pression atmosphérique, sauf indication contraire.

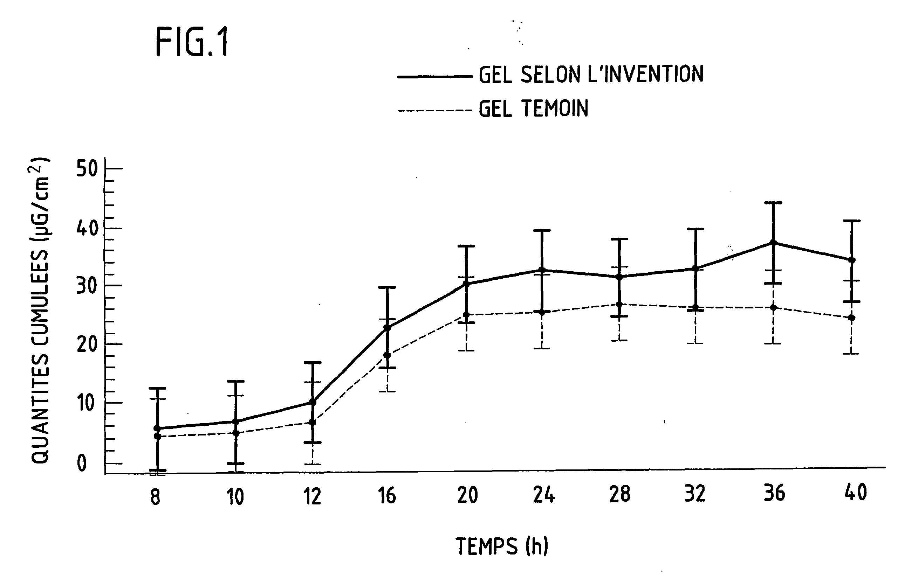

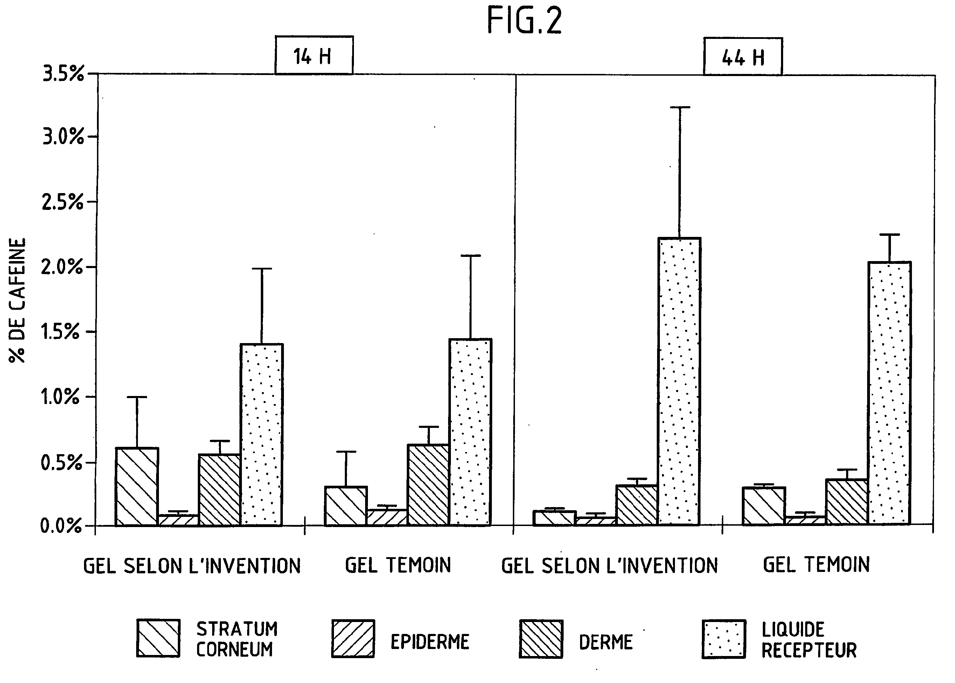

L'exemple 15 est illustré par les figures 1 et 2, qui représentent respectivement :
- figure 1 : la cinétique de pénétration de la caféine,
- figure 2 : la répartition de la caféine au sein des assises cutanées après 14 heures et 44 heures de contact.

### Exemple 1 : Préparation d'un extrait de l'algue Laminaria flexicaulis riche en alginate sous forme de gel:

On utilise ici 200g d'algue Laminaria flexicaulis. On peut utiliser soit l'algue entière, soit une partie de celle-ci, en particulier les thalles.

Les algues sont tout d'abord rincées pendant 1 à 3 heures dans de l'eau froide afin d'éliminer les substances solubles telles que les iodures. Après avoir été lavées, les algues sont séchées puis déchiquetées avant d'être blanchies. Pour cela, on utilise un procédé de blanchiment classique qui consiste en l'utilisation d'eau de Javel à 0,1% et/ou d'eau oxygénée à 3%. Les algues sont plongées pour une durée variable de 3 à 7 jours dans des bains d'eau de Javel et/ou des bains d'eau oxygénée. A ce stade de la préparation, le produit obtenu est rincé abondamment à l'eau froide puis broyé finement dans de l'eau distillée à l'aide d'une turbine de type Turrax, à grande vitesse, jusqu'à l'obtention d'un gel fluide. Pour ce broyage, la proportion de produit sec dans l'eau est comprise entre 1 et 5 litres d'eau pour 200 g d'algues Laminaria flexicaulis. L'extrait ainsi obtenu, sous forme de pâte ou de gel est incolore ou très légèrement beige et translucide. Il est essentiellement constitué d'alginate et de cellulose.

Cet extrait est ensuite avantageusement séché selon des procédés bien connus de l'homme de l'art. L'extrait sec d'algue ainsi obtenu est prêt à être incorporé pour la préparation d'un produit à effet tenseur selon l'invention.

### Exemple 2 : Préparation d'un extrait de sorbes riche en sorbitol:

Dans un premier temps, on broie 50g de sorbes entières issues de Sorbus aucuparia puis on additionne 50mL d'eau distillée que l'on porte à ébullition 10 min afin d'obtenir une réduction d'environ 60 mL. On filtre alors sous pression (1 à 10 kg/cm²) à travers une gaze stérile (2 à 5 strates, de préférence 3) ou un tamis (100 à 200µm) en verre ou inox fritté. On obtient ainsi un jus de couleur rose qui peut être utilisé tel que ou bien concentré selon le mode d'utilisation souhaité.

L'extrait de sorbes ainsi obtenu est prêt à être incorporé pour la préparation d'un produit à effet tenseur selon l'invention.

On donnera ci-après divers exemples de formulations de produits notamment à effet tenseur de la surface de la peau qui constituent un produit de base pour la préparation d'une composition cosmétique selon l'invention.

### Exemple 3 : Mélange selon l'invention à effet tenseur très raide

| | |
|---|---|
| Alginate de Na | 43,00 % |
| CMC | 6,75% |
| Concentré selon l'exemple 2 à 70% de sorbitol | 47,00 % |
| APV | 3% |
| Cellulose | 0,25 % |

### Exemple 4 : Mélange selon l'invention à effet tenseur moyen

| | |
|---|---|
| Extrait sec d'algue selon l'exemple 1 | 50% |
| (Extrait à 85% d'alginate et 5% de cellulose) | |
| Concentré selon l'exemple 2 à 70% de sorbitol | 46% |
| APV | 4% |

### Exemple 5 : Mélange selon l'invention à effet tenseur souple

| | |
|---|---|
| Alginate de Na | 22% |
| CMC | 4% |
| Concentré selon l'exemple 2 à 70% de sorbitol | 65% |
| APV | 9% |

### Exemple 6 : Mélange à effet tenseur contenant de la PVP*

| | |
|---|---|
| Alginate de Na | 42 % |
| CMC | 4% |
| Mannitol | 49% |
| PVP | 5% |

| | |
|---|---|
| *(non conforme à l'invention) | |

### Exemple 7 : Mélange à effet tenseur selon l'invention

| | |
|---|---|
| Sorbitol | 64,68 % |
| Alginate | 22 % |
| APV | 9,15 % |
| CMC | 4,17 % |

### Exemple 8 : Mélange tenseur selon l'invention

| | |
|---|---|
| Sorbitol | 40 % |
| Alginate de sodium | 50 % |
| APV | 6% |
| CMC | 4% |

### Exemple 9 : Mélange tenseur selon l'invention

| | |
|---|---|
| Sorbitol | 46,05 % |
| Alginate de sodium | 46,05 % |
| APV | 3,8 % |
| CMC | 4,1 % |

### Exemple 10 : Mélange tenseur selon l'invention

| | |
|---|---|
| Sorbitol | 47 % |
| Alginate | 43 % |
| APV | 3% |
| CMC | 7% |

### Exemple 11 : Composition cosmétique utilisant le produit à effet tenseur de la surface de la peau selon l'invention à base d'alginate de sodium et de sorbitol

| | |
|---|---|
| Mélange selon l'exemple 5 | 8% |
| CMC | 0,33% |
| Phénoxyéthanol et paraben | 0,5% |
| Parahydroxybenzoate de méthyle | 0,1% |
| Eau purifiée | qsp100 |

### Exemple 12 : Composition cosmétique selon l'invention sous forme de gel

| | |
|---|---|
| Mélange à effet tenseur de l'exemple 7 | 2,8 % |
| Excipient gélifié aqueux + conservateur + parfum | qsp 100 |

### Exemple 13 : Composition cosmétique anti-rides à effet tenseur selon l'invention, sous forme d'émulsion

Cette émulsion est constituée de :

| | |
|---|---|
| Mélange à effet tenseur de l'exemple 7 | 0,8 % |
| Extrait de tormentille | 0,5 % |
| Phase grasse | 19 % |
| Phase aqueuse gélifiée + conservateur + parfum | qsp 100 |

Cette composition procure un effet tenseur immédiat et prolongé, ce qui permet un lissage de la peau par effet mécanique. De plus, grâce à l'extrait de plante actif pour atténuer les rides, cette composition permet simultanément une atténuation des rides plus durable encore par un effet biologique.

Cet effet biologique est en outre renforcé par « l'effet réservoir » procuré par la présence dans la composition du mélange selon l'invention.

### Exemple 14. Evaluation de l'effet lissant immédiat des rides in vivo :

### 1. Méthode :

Une analyse d'empreintes des rides de la peau est effectuée par une méthode sans contact à projection de franges.

Cette méthode est couramment utilisée dans diverses industries, notamment pour mesurer la rugosité d'une surface. Cette technique est, par exemple, décrite dans la publication intitulée « Optical roughness measurements with fringe projections » de Robert WINDECKER, Stefan FRANZ, Hans J. TIZIANI, Applied optics, 1999, vol. 38, n°13, pp. 2837-2842.

Des franges d'interférences sont projetées directement sur la peau à l'aide d'une optique spécifique et sont capturées comme une image par une caméra numérique. A partir des images de projection de franges, une image de phase est calculée et l'altitude de chaque point est déterminée.

Un logiciel d'acquisition permet d'obtenir des mesures 2D, 3D de la peau et de déterminer les paramètres de rugosité sur un profil, la profondeur et le volume des rides.

### 2. Protocole :

Une zone au niveau d'une patte d'oie d'un volontaire n'ayant appliqué aucun produit au niveau du visage depuis la veille au soir est déterminée et repérée. Une acquisition d'image est alors effectuée.

Sur cette même zone, une dose de 2µL par cm² de composition selon l'invention est appliquée.

Une nouvelle image de cette même zone est ensuite acquise après 45 minutes et 4 heures.

Un repositionnement automatique permet la ré-identification précise de la zone de mesure à chaque temps.

Les images sont comparées entre elles et trois paramètres particuliers sont étudiés : la rugosité moyenne Ra, l'écart minimum entre la pic le plus haut et la vallée la plus profonde Rt et la valeur moyenne des maxima Rz.

La rugosité moyenne Ra se définit comme le rapport de la surface intégrée autour de la valeur moyenne sur la longueur de peau balayée.

### 3. Résultats :

La composition selon l'exemple 13 de l'invention appliquée sur la peau diminue la rugosité moyenne, Ra, de celle-ci de 13% dès 45 minutes d'application et de 21% au bout 4 heures d'application.

Les mêmes essais montrent une diminution de l'écart minimum entre le pic le plus haut et le creux le plus profond, Rt, de 18% dès 45 minutes d'application et de 14% au bout 4 heures d'application. De la même manière, la valeur moyenne des différents maxima, Rz, diminue de 14% dès 45 minutes d'application et de 19% au bout 4 heures d'application.

Ces résultats démontrent un effet lissage des rides, grâce à l'application de la composition tenseur selon l'invention, immédiat.

### Exemple 15. Influence du mélange selon l'invention sur la cinétique de pénétration de la caféine.

### 1. But de l'étude :

Cette étude est réalisée, *in vitro,* sur peau humaine, préalablement conservée par congélation, dans le but d'évaluer l'influence du mélange selon l'invention sur la pénétration et la répartition de la caféine au sein des assises cutanées. Les propriétés occlusives d'un gel selon l'invention amènent la recherche d'un éventuel effet patch. Cette étude est réalisée en comparaison avec un gel de Carbopol.

### 2. Méthodologie :

Le principe d'étude est basé sur l'évaluation de la diffusion d'un principe actif à travers la peau humaine décongelée, montée sur cellules de Franz.

Ces cellules sont constituées de deux éléments en verre délimitant un compartiment supérieur et un compartiment inférieur, elles présentent une surface d'exposition de 3,8 cm. La peau est placée entre ces deux éléments, *stratum corneum* vers le haut. Le principe actif déposé sur la peau traverse celle-ci et il est recueilli, au niveau du compartiment inférieur, solubilisé dans une solution dite réceptrice, constituée d'un mélange tampon phosphate + nitrure de Na (0,1%).

La méthode fait référence aux directives du COLIPA établies dans le domaine de la modélisation *in vitro* de l'absorption cutanée (c.f. Validation of the pig ear skin model for in vitro percutaneous absorption : inter-laboratory reproducibility studies. CM. Vincent & coll., Perspectives in Percutaneous Pénétration, Vol. 7A, 2000).

1 mL de formulation, soit 5 mg de principe actif (dose infinie), sont déposés aléatoirement sur chaque expiant. L'étude est réalisée en condition non occlusive, afin de favoriser le séchage du polymère et de permettre ainsi, un éventuel effet patch. Les expériences sont répétées à raison de 6 cellules de Franz par produit.

Les formulations testées sont les suivantes :

| **Matières premières en %** | **Gel Témoin** | **Gel selon l'invention** |
|---|---|---|
| Caféine | 0,5 | 0,5 |
| Stachyose | 1,0 | 1,0 |
| Mélange de l'exemple 7 | - | 1,2 |
| Ethanol | 15,0 | 15,0 |
| Carbopol 940 | 0,5 | 0,5 |
| Triéthanol amine | 0,5 | 0,5 |
| Phénoxétol | 0,2 | 0,2 |
| Eau | 82,3 | 81,1 |

### a) cinétique de pénétration :

La cinétique de pénétration est établie par prélèvements du liquide de survie entre 8 et 40 heures d'application.

### b) Compartimentation :

Au terme de 14 h et 44 h de contact, la moitié des cellules est démontée, les biopsies subissent un traitement de lavage durant lequel l'excédent d'émulsion à la surface cutanée est lavé à l'aide de "coton tige". Les cotons tiges ainsi que la partie supérieure de la cellule sont immergés dans 10 mL de mélange méthanol/eau à 50/50 pendant 30 min. sous agitation. La solution est ensuite filtrée et diluée au 1/10 dans une solution méthanol / eau (50/50) avant dosage.
Le *Stratum corneum* est recueilli par 8 exfoliations successives au moyen d'une bande adhésive, en appliquant une pression de 300 g/cm² pendant 12 s. La caféine est extraite par solubilisation dans 2 mL de méthanol/eau sous agitation pendant 30 min.
L'épiderme est séparé du derme par chauffage à 60°C pendant 12 s., puis séparé du derme à l'aide de pinces fines. La caféine est extraite par contact avec 2 mL de méthanol/eau durant 24 h.
Le derme est placé dans 2 mL de méthanol/eau, pendant 24 h.
Les suspensions épidermiques et dermiques sont agitées pendant 2 h et filtrées à 0,45 µm avant dosage.
Le dosage de la caféine est réalisé par HPLC.

### 3. Expression des résultats :

La quantité de principe actif recueilli au sein des divers compartiments biologiques étudiés est exprimée en µg/mL.

La cinétique de pénétration, en µg/cm² par unité de temps, est exprimée en fonction de l'intervalle de confiance, IC_{95%} où l'écart type résiduel est calculé par la méthode ANOVA sur l'ensemble des essais.

La compartimentation est le pourcentage de caféine absorbée en fonction du temps : pour chaque compartiment étudié, la proportion de caféine absorbée est calculée à partir des quantités recueillies par rapport aux quantités appliquées.

### 4. Résultats :

### a) Limite de solubilité de la caféine :

Afin d'éviter tout risque de sous-estimation de l'absorption cutanée, la concentration de principe actif dans le liquide récepteur doit être inférieure à 10% de la concentration limite de solubilité (Guidelines for percutaneous absorption, Colipa, Nov 95).

A 37°C, la concentration limite de solubilité de la caféine dans le liquide récepteur est de 16 mg/mL. La concentration maximum de caféine recueillie au cours de l'étude est inférieure à 23 µg/mL; les conditions expérimentales sont donc correctes.

### b) Cinétique de pénétration, in vitro, de la caféine :

La figure 1 indique les quantités de caféine recueillies dans le liquide récepteur en fonction du temps.

La cinétique de pénétration de la caféine, à partir de la formulation contenant la composition selon l'invention est plus élevée qu'avec le gel témoin à base de Carbopol. A 40 h , la concentration de caféine (33,6 µg/cm²) présente au niveau du liquide récepteur après application du gel selon l'invention est supérieure à celle dosée après application du gel témoin (24,0 µg/cm²).

En séchant, le complexe gélifiant selon l'invention provoque un léger effet occlusif qui favorise l'absorption percutanée du principe actif.

Une analyse de variance (ANOVA), complétée par un test de classification de Newman-Keuls, a été réalisée afin de vérifier s'il existe une différence significative entre les quantités absorbées après 12 h, 40 h et tous temps confondus.

L'étude statistique réalisée montre que, dans les conditions expérimentales, l'addition du mélange selon l'invention dans un gel favorise, de façon significative, la pénétration de la caféine dans le revêtement cutané.

### 5. Etude compartimentale :

La proportion de caféine absorbée est calculée à partir des quantités recueillies par rapport aux quantités appliquées.

La répartition de la caféine au sein des assises cutanées après 14 et 44 h de contact est donnée dans le tableau ci-dessous où les résultats sont exprimés en %.

| | Après 14 heures | | Après 44 heures | |
|---|---|---|---|---|
| | Gel de l'invention | Gel témoin | Gel de l'invention | Gel témoin |
| STRATUM CORNEUM | 0,65 | 0,31 | 0,10 | 0,26 |
| EPIDERME | 0,10 | 0,11 | 0,04 | 0,04 |
| DERME | 0,56 | 0,62 | 0,30 | 0,32 |
| LIQUIDE RECEPTEUR | 1,41 | 1,42 | 2,19 | 2,00 |

L'analyse des résultats présentés dans le tableau ci-dessus et sur la figure 2 démontre une meilleure accumulation de la caféine dès 14 h au niveau de la couche cornée après application du gel selon l'invention, traduisant ainsi une amélioration de l'effet réservoir du *stratum corneum.* Cette observation est représentative d'un effet patch.

### 6. Conclusions :

L'effet patch du gel selon l'invention se traduit expérimentalement par :
- Une tendance à l'amélioration de l'effet réservoir du stratum *corneum* vis à vis de la caféine, favorisant ainsi sa biodisponibilité.
- Une cinétique de pénétration de la caféine significativement plus élevée après addition du gel selon l'invention.

## Revendications

1. Mélange à effet tenseur de la peau constitué de :
- 10 à 80 % en poids d'un polysaccharide ayant un poids moléculaire inférieur à 600 000 Daltons, de préférence inférieur à 400 000 Daltons et de préférence encore inférieur à 200 000 Daltons,
- 15 à 75 % en poids de sorbitol,
- 3 à 15 % en poids d'alcool polyvinylique ou de polyvinylpyrrolidone,
- 0 à 25 % en poids de cellulose ou d'un dérivé de cellulose, tel que la carboxyméthylcellulose ou l'hydroxyméthylcellulose.

2. Mélange selon la revendication 1, **caractérisé en ce que** ledit polysaccharide est une pectine, un carraghénane, un galactomannane, un xanthane ou un alginate.

3. Mélange selon l'une des revendications 1 ou 2, **caractérisé en ce que** ledit polysaccharide est un alginate, en particulier un alginate de propylène glycol, ou un alginate sous forme d'acide ou de sel, en particulier un alginate de sodium.

4. Mélange selon la revendication 3, **caractérisé en ce que** ledit alginate se présente sous forme d'un extrait d'algue, en particulier un extrait d'algue de la famille des Laminariacae, de préférence un extrait d'algue de Laminaria digitata, ou de Laminaria flexicaulis, ou encore de Laminaria saccharina.

5. Mélange selon l'une des revendications 1 à 4, **caractérisé en ce que** la concentration en polysaccharide est comprise entre 20 % et 55 % en poids.

6. Mélange selon l'une des revendications 1 à 5, **caractérisé en ce que** le sorbitol se présente sous la forme d'un extrait de plante, en particulier un extrait de sorbes, tel qu'un extrait de sorbes de Sorbus aucuparia, de sorbes de Sorbus domestica ou de sorbes de Sorbus aria.

7. Mélange selon l'une des revendications 1 à 6, **caractérisé en ce que** le sorbitol est à une concentration comprise entre 40 et 65 % en poids.

8. Mélange selon l'une des revendications 1 à 7, **caractérisé en ce qu'**il contient un extrait de Laminaria flexicaulis et un extrait de sorbes, en particulier de sorbes de Sorbus aucuparia.

9. Mélange selon l'une des revendications 1 à 8, **caractérisé en ce que** la concentration en polysaccharide est supérieure à la concentration en sorbitol.

10. Mélange selon l'une des revendications 1 à 8, **caractérisé en ce que** la concentration en sorbitol est supérieure à la concentration en polysaccharide.

11. Mélange selon l'une des revendications 1 à 10, **caractérisé en ce qu'**il contient de 3 à 10 % en poids d'alcool polyvinylique ou de polyvinylpyrrolidone.

12. Mélange selon l'une des revendications 1 à 11, **caractérisé en ce qu'**il contient de 1 à 8 % en poids de cellulose ou d'un dérivé de cellulose.

13. Mélange selon l'une des revendications 1 à 12, **caractérisé en ce qu'**il est constitué de :
- 10 à 80 %, de préférence 20 à 55 %, en poids d'alginate,
- 15 à 75 %, de préférence 40 à 65 %, en poids de sorbitol,
- 3 à 15 %, de préférence 3 à 10 %, en poids d'alcool polyvinylique ou de polyvinylpyrrolidone,
- 0 à 25 %, de préférence 1 à 8 %, en poids de carboxyméthylcellulose.

14. Composition cosmétique, **caractérisée en ce qu'**elle contient de 0,2 à 90 %, de préférence de 0,4 à 70 %, de préférence encore de 0,4 à 5 % et plus préférentiellement de 1 à 3 %, en poids par rapport au poids total de ladite composition d'un mélange tel que défini dans l'une des revendications 1 à 13.

15. Composition cosmétique selon la revendication 14, **caractérisée en ce qu'**elle contient en outre au moins un agent biologiquement actif, tel qu'un agent hydratant, un agent anti-ride, un agent anti-oxydant, un agent anti-radicalaire, un agent réparateur des effets destructeurs des rayons ultra-violets ou un agent amincissant tel que de la caféine.

16. Composition cosmétique selon l'une des revendications 14 ou 15, **caractérisée en ce qu'**elle contient en outre un parfum, une matière colorante cosmétiquement acceptable, et/ou un agent protecteur des rayons ultra-violets UVA ou UVB, notamment un filtre ou un nano pigment, tel qu'un oxyde de zinc ou de titane.

17. Composition cosmétique selon l'une des revendications 14 à 16, **caractérisée en ce qu'**elle se présente sous la forme d'un gel, d'une lotion, d'un sérum, d'une suspension, d'une émulsion huile-dans-eau ou eau-dans-huile, ou sous forme solide, par exemple sous forme d'un stick ou d'un patch, ou sous forme sèche, par exemple sous forme d'une poudre.

18. Procédé non-thérapeutique de soin cosmétique, **caractérisé en ce qu'**il comprend l'application topique, sur les zones de la peau concernées d'une quantité efficace d'une composition cosmétique, telle que définie dans l'une quelconque des revendications 14 à 17, pour obtenir un effet de lissage de la surface de la peau.

19. Procédé selon la revendication 18, **caractérisé en ce que**, ultérieurement à l'application de ladite composition cosmétique, et en cas de diminution de l'effet tenseur de la surface de la peau, on réalise une humidification des zones de la peau concernées ayant reçu au moins une première application de ladite composition cosmétique, afin de restaurer ledit effet tenseur, notamment par pulvérisation d'eau ou d'une préparation aqueuse cosmétiquement acceptable.

20. Utilisation cosmétique d'un mélange tel que défini dans l'une des revendications 1 à 13 comme agent cosmétique pour favoriser la pénétration dans la peau d'un agent biologiquement actif.

## Claims

1. A mixture, having a skin-tightening effect, which is made up of :
- 10 to 80 % by weight of a polysaccharide having a molecular weight of less than 600,000 Daltons, preferably less than 400,000 Daltons and more preferably less than 200,000 Daltons,
- 15 to 75 % by weight of sorbitol,
- 3 to 15 % by weight of poly(vinyl alcohol) or polyvinylpyrrolidone, and
- 0 to 25 % by weight of cellulose or of a derivative of cellulose, such as carboxymethylcellulose or hydroxymethylcellulose.

2. The mixture according to claim 1, **characterised in that** said polysaccharide is a pectin, a carrageenan, a galactomanane, a xanthan, or an alginate.

3. The mixture according to one of claims 1 or 2, **characterised in that** said polysaccharide is an alginate, particularly an alginate of propylene glycol, or an alginate in the form of an acid or a salt, particularly a sodium alginate.

4. The mixture according to claim 3, **characterised in that** said alginate is in the form of an extract of alga, particularly an extract of alga of the family of Laminariacae, preferably an extract of Laminaria digitata alga, or of Laminaria flexicaulis alga, or even of Laminaria saccharina alga.

5. The mixture according to one of claims 1 to 4, **characterised in that** the concentration of polysaccharide is between 20 % and 55 % by weight.

6. The mixture according to one of claims 1 to 5, **characterised in that** the sorbitol is in the form of a plant extract, particularly an extract of sorbs, such as a Sorbus aucuparia sorb extract, a Sorbus domestica sorb extract, or a Sorbus aria sorb extract.

7. The mixture according to one of claims 1 to 9, **characterised in that** sorbitol is at a concentration of between 40 and 65 % by weight.

8. The mixture according to one of claims 1 to 7, **characterised in that** it contains a Laminaria flexicaulis extract and an extract of sorbs, particularly of Sorbus aucuparia sorbs.

9. The mixture according to one of claims 1 to 8, **characterised in that** the concentration of polysaccharide is greater than the concentration of sorbitol.

10. The mixture according to one of claims 1 to 8, **characterised in that** the concentration of sorbitol is greater than the concentration of polysaccharide.

11. The mixture according to one of claims 1 to 10, **characterised in that** it contains 3 to 10 % by weight of poly(vinyl alcohol) or polyvinylpyrrolidone.

12. The mixture according to one of claims 1 to 11, **characterised in that** it contains 1 to 8 % by weight of cellulose or of a derivative of cellulose.

13. The mixture according to one of claims 1 to 12, **characterised in that** it is made up of :
- 10 to 80 %, preferably 20 to 55 %, by weight of alginate,
- 15 to 75 %, preferably 40 to 65 %, by weight of sorbitol,
- 3 to 15 %, preferably 3 to 10 %, by weight of poly(vinyl alcohol) or polyvinylpyrrolidone, and
- 0 to 25 %, preferably 1 to 8 %, by weight of carboxymethylcellulose.

14. A cosmetic composition, **characterised in that** it contains 0.2 to 90 %, preferably 0.4 to 70 %, more preferably 0.4 to 5 %, and more preferentially 1 to 3 %, by weight with respect to the total weight of said composition, of a mixture as defined in one of claims 1 to 13.

15. The cosmetic composition according to claim 14, **characterised in that** it further contains at least one biologically active agent, such as a moisturising agent, an anti-wrinkle agent, an anti-oxidising agent, an anti-radical agent, an agent which repairs the destructive effects of ultra violet rays, or a slimming agent such as caffeine.

16. The cosmetic composition according to one of claims 14 or 15, **characterised in that** it further contains a perfume, a cosmetically acceptable colouring material, and/or an agent which protects against UVA or UVB ultra violet rays, notably a filter or a nano pigment, such as a zinc oxide or a titanium oxide.

17. The cosmetic composition according to one of claims 14 to 16, **characterised in that** it is in the form of a gel, a lotion, a serum, a suspension, an oil-in-water or water-in-oil emulsion, or in solid form, for example in the form of a stick or a patch, or in dry form, for example in the form of a powder.

18. A non-therapeutic method of cosmetic care, **characterised in that** it comprises applying topically, on the zones of the skin concerned, an effective amount of a cosmetic composition, as defined in any one of claims 14 to 17, to obtain a smoothing effect on the surface of the skin.

19. The method according to claim 18, **characterised in that** subsequent to the application of said cosmetic composition, and in case of reduction of the tightening effect on the surface of the skin, a moisturisation is carried out of the zones of the skin concerned having received at least one first application of said cosmetic composition, so as to restore said tightening effect, notably by spraying water or a cosmetically acceptable aqueous preparation.

20. Cosmetic use of a mixture as defined in one of claims 1 to 13 as cosmetic agent for promoting the penetration in the skin of a biologically active agent.

## Patentansprüche

1. Gemisch mit Hautstraffungswirkung, bestehend aus:
- 10 - 80 Gew.-% eines Polysaccharids mit einem Molekulargewicht unter 600 000 Dalton, vorzugsweise unter 400 000 Dalton und weiter bevorzugt unter 200 000 Dalton,
- 15 - 75 Gew.-% Sorbitol
- 3-15 Gew.-% Polyvinylalkohol oder Polyvinylpyrrolidon,
- 0-25 Gew.-% Cellulose oder von einem Cellulosederivat wie Carboxymethylcellulose oder Hydroxymehtylcellulose.

2. Gemisch nach Anspruch 1, **dadurch gekennzeichnet, daß** das Polysaccharid ein Pektin, ein Carraghenan, ein Galactomannan, ein Xanthan oder ein Alginat ist.

3. Gemisch nach Anspruch 1 oder 2, **dadurch gekennzeichnet, daß** das Polysaccharid ein Alginat, insbesondere ein Propylenglycolalginat oder ein Alginat in Form einer Säure oder eines Salzes, insbesondere ein Natriumalginat ist.

4. Gemisch nach Anspruch 3, **dadurch gekennzeichnet, daß** das Alginat in Form eines Algenextraktes vorliegt, insbesondere eines Extraktes einer Alge aus der Familie der Laminariaceae, vorzugsweise eines Extraktes aus Laminaria digitata oder Laminaria flexicaulis oder auch Laminaria saccharina.

5. Gemisch nach einem der Ansprüche 1 bis 4, **dadurch gekennzeichnet, daß** die Polysaccharidkonzentratrion zwischen 20 und 55 Gew.-% liegt.

6. Gemisch nach einem der Ansprüche 1 bis 5, **dadurch gekennzeichnet, daß** das Sorbitol in Form eines Pflanzenextraktes vorliegt, insbesondere eines Vogelbeerextraktes, wie einem Extrakt von Vogelbeeren von Sorbus aucuparia, von Vogelbeeren von Sorbus domestica oder von von Sorbus aria.

7. Gemisch nach der Ansprüche 1 bis 6, **dadurch gekennzeichnet, daß** das Sorbitol bei einer Konzentration zwischen 40 und 65 Gew.-% vorliegt.

8. Gemisch nach einem der Ansprüche 1 bis 7, **dadurch gekennzeichnet, daß** es einen Extrakt von Laminaria flexicaulis und ein Vogelbeerextrakt, insbesondere von Vogelbeeren von Sorbus aucuparia enthält.

9. Gemisch nach einem der Ansprüche 1 bis 8, **dadurch gekennzeichnet, daß** die Polysaccharidkonzentration über der Sorbitolkonzentration liegt.

10. Gemisch nach einem der Ansprüche 1 bis 8, **dadurch gekennzeichnet, daß** die Sorbitolkonzentration über der Polysaccharidkonzentration liegt.

11. Gemisch nach einem der Ansprüche 1 bis 10, **dadurch gekennzeichnet, daß** es 3 bis 10 Gew.-% Polyvinylalkohol oder Polyvinylpyrrolidon enthält.

12. Gemisch nach einem der Ansprüche 1 bis 11, **dadurch gekennzeichnet, daß** es 1 bis 8 Gew.-% Cellulose oder eines Cellulosederivats enthält.

13. Gemisch nach einem der Ansprüche 1 bis 12, **dadurch gekennzeichnet, daß** es besteht aus:
- 10 bis 80 Gew.-%, vorzugsweise 20 bis 55 Gew.-% Alginat
- 15 bis 75 Gew.-%, vorzugsweise 40 bis 65 Gew.-% Sorbitol
- 3 bis 15 Gew.-%, vorzugsweise 3 bis 10 Gew.-% Polyvinylalkohol oder Polyvinylpyrrolidon,
- 0 bis 25 Gew.-%, vorzugsweise 1 bis 8 Gew.-% Carboxymethylcelluslose.

14. Kosmetische Zusammensetzung, **dadurch gekennzeichnet, daß** sie 0,2 bis 90 Gew.-%, vorzugsweise 0,4 bis 70 Gew.-%, weiter bevorzugt 0,4 bis 5 Gew.-% und bevorzugter 1 bis 3 Gew.-% im Verhältnis zum Gesamtgewicht der Zusammensetzung von einem Gemisch wie definiert in einem der Ansprüche 1 bis 13 enthält.

15. Kosmetische Zusammensatzung nach Anspruch 14, **dadurch gekennzeichnet, daß** sie außerdem wenigstens ein biologisch wirksames Mittel enthält wie ein Feuchtigkeit spendendes Mittel, ein Antifaltenmittel, ein Antioxidationsmittel, ein Antiradikalmittel, ein Ausbesserungsmittel der Zerstörungswirkungen der UV-Strahlen oder ein Schlankheitsmittel wie Koffein.

16. Kosmetische Zusammensetzung nach Anspruch 14 oder 15, **dadurch gekennzeichnet, daß** sie außerdem ein Parfum, ein kosmetisch verträgliches Färbungsmittel und/oder ein UVA- oder UVB-UV-Strahlenschutzmittel umfaßt, insbesondere einen Filter oder ein Nanopigment wie ein Zink- oder Titanoxid.

17. Kosmetische Zusammensetzung nach einem der Ansprüche 14 bis 16, **dadurch gekennzeichnet, daß** sie in Form eines Gels, einer Lotion, eines Serums, einer Suspension, einer Öl-in-Wasser-Suspension oder Wasser-in-Öl-Suspension oder in fester Form, zum Beispiel in Form eines Stäbchens oder eines Pflasters oder in trockener Form, zum Beispiel in Form eines Pulvers vorliegt.

18. Nicht therapeutisches Verfahren zur kosmetischen Pflege, **dadurch gekennzeichnet, daß** es die topische Anwendung auf die betreffenden Hautzonen einer wirksamen Menge einer kosmetischen Zusammensetzung wie definiert in einem der Ansprüche 14 bis 17 umfaßt, um eine Glättungswirkung der Hautoberfläche zu erhalten.

19. Verfahren nach Anspruch 18, **dadurch gekennzeichnet, daß** man nach der Anwendung der kosmetischen Zusammensetzung und im Fall einer Verminderung der Straffungswirkung der Hautoberfläche eine Befeuchtung der betreffenden Hautbereiche durchführt, die wenigstens eine erste Anwendung der kosmetischen Zusammensetzung empfangen haben, um die Straffungswirkung wiederherzustellen, insbesondere durch Zerstäubung von Wasser oder einer kosmetisch verträglichen wäßrigen Präparation.

20. Kosmetische Verwendung eines Gemisches wie definiert in der Ansprüche 1 bis 13 als kosmetisches Mittels, um das Eindringen eines biologisch aktiven Mittels in die Haut zu begünstigen.
